# EUROPEAN PATENT SPECIFICATION

(11) **EP 1 921 091 B1**
(45) Date of publication and mention of the grant of the patent: **24.06.2009**
(21) Application number: 07021621.3
(22) Date of filing: 07.11.2007
(51) Int. Cl.: C08B 37/00, A61K 31/728, A61K 31/192

(54) **Process for the preparation of esters of the diacerein with hyaluronic acid and pharmaceutical preparations containing them**
Verfahren zur Zubereitung von Estern des Diacereins mit Hyaluronsäure und pharmazeutische Präparate enthaltend dieselben
Procédé pour la préparation d'esters de diacéréine avec de l'acide hyaluronique et préparation pharmaceutiques les contenant

(30) Priority: 08.11.2006 IT MI20062134
(43) Date of publication of application: 14.05.2008
(73) Proprietor: CBB Net S.A., 6900 Lugano (CH)
(72) Inventor: Borsa, Massimiliano, 20190 Vimodrone/MI (IT); Merighi, Roberto, 45025 Fratta Polesine/RO (IT)
(74) Representative: Beneduce, Gianna

(56) References cited:
- EP-A- 0 809 995
- WO-A-03/007921
- WO-A-20/05085293

## Description

### State of the art

The hyaluronic acid is a disaccharide polymer composed of D-glucuronic acid and N-acetylglucosamine, distributed in the body as intracellular tissue and in the fluids, such as the vitreous humour and synovial liquid. In EU hyaluronic acid sodium salt is used for intra-articular and intra-ocular administration. The wide interval in molecular weight and consequent intrinsic viscosity allow its use in the healing of wounds, in ocular operations, in osteo-arthritis of the large joints (knee).

Hyaluronic acid, solution 20-30 mg/vial, has been used for over 15 years for intra-articular administration with the aim of replacing the hyaluronic acid normally present in the joint, which, with the progression of the illness undergoes to a more rapid depolymerisation with consequent increase of severity of the pathology.

The therapeutic value of its intra-articular administration is well documented (J.Rheumatol. 25:2203-2212, 1998; ibid 26:1216,1999; ibid 38:602-607, 1999) and the efficacy is substantially attributed to the "lubrication" of the joint (Arch.Int.Med. 162:245-7, 2002). These results suggest the intra-articular administration as a "supplementation" of high-viscosity acid and the regulating state of the product was changed in the last years from medicinal drug product to "medical device".

Diacerein is a drug authorised for oral use in various European countries for the treatment of osteoarthritis, because it is able of inhibiting the synthesis of interleukin-1 (IL-1) and the production of nitrous oxides (NO), induced by the same IL-1, which are among the agents responsible for cartilaginous degeneration. The aetiological action of the diacerein was confirmed "in vivo" and "in vitro" (Presse Med. 2004 May 22;33(9 Pt 2):S10-2; Biorheology 2002;39(1-2):277-85; Arthritis Rheum. 2001 Nov; 44(11):2539-47; J.Rheumatol. 2001 Apr; 28(4):814-24; Arthritis Rheum. 2000 Oct; 43(10):2339-48; Osteoarthritis Cartilage 2000 May; 8(3):186-96; Clin.Exp.Rheumatol. 2003 Mar-Apr; 21(2):171-7; Pharmacol.Toxicol.,2002 July; 91(1):22-8; Osteoarthritis Cartilage, 2001 Apr; 9(3):257-63).

Diacerein has a limited bioavailability: its intestinal metabolism produces metabolites with a laxative action; it is a pro-drug of rhein, that penetrates rapidly into the synovial liquid at low concentrations (1-10 mmol/L ) and it is rapidly eliminated. These characteristics - low absorption, hydrolysis in the stomach into rhein, anthraquinonic metabolites with a laxative effect, rapid elimination - give reason of the poor tolerability of oral diacerein therapy and of the advantage of using local administration in the target area.

Intra-articular administration of diacerein is difficult to realize due to the lack of solubility of the drug in a carrier compatible with the synovial liquid, and because of its permanence time in the joint, which has been shown to be too short for an effective block of the IL-1 synthesis.

International Patent Application WO 2005/085293 describes esters of rhein with hyaluronic acid, prepared by warm reacting hyaluronic acid with rhein chloride and following purification by ultra filtration or dialysis.

This method of synthesis, due to the stressed conditions of reaction, causes the formation of a number of red purple coloured by-products of rhein, which resulted very difficult to remove, either by ultra filtration or by dialysis, for the occurrence of secondary reactions with hyaluronic acid itself. After ultrafiltration or dialysis, the final compound appears as a red powder, with a negligible content of hydrolysable rhein (less that 1%), with a solubitily in water or saline less that 1 mg/ml, useless for the local administration.

It the synthesis of hyaluronic acid and rhein chloride is performed in controlled mild conditions (30-40°C, hydrophobic solvent with moisture less than 1%, absence of Lewis bases), rhein remains simply "trapped" in the hyaluronic acid structure, and it is washed during dialysis or ultrafiltration. The final compound after drying appears almost white but it contains less than 0,1% of active drug.

In said Patent Application WO 2005/085293 there are, generically mentioned, but not exemplified, other possible esters of hyaluronic acid with acyl derivatives of rhein, including the diacetyl derivative or diacerein. As to the preparation of the diacerein ester of the hyaluronic acid the method described in WO2005/08529 proved to be unsuccessful: at the process conditions, the diacerein is hydrolized into rhein and other derivatives, with subsequent secondary reactions and by-products formation.

### Description of the invention

The object of the present invention is a process for the preparation of stable esters of diacerein with hyaluronic acid having a high purity level, which are endowed of an interesting anti-arthritis activity free from side effects and suitable for intra-articular administration. The hyaluronic acid used in these esters is preferably of a molecular weight in the range from 100,000 to 1,500,000 Da.

The esters of hyaluronic acid and diacerein, prepared according to the method of the invention, display the following characteristics:
- they do not contain detectable impurities of diacerein derivatives, such as non-linked diacerein, rhein and their derivatives;
- they are white or straw-white in colour, the intensity of which depends on the diacerein content;
- they can be administered in physiological solution or in aqueous organic solution, as saline-glycerol or saline-PEG solution;
- they allow, when administered in the joint, a residence time of diacerein that is inversely proportional to the depolymerization rate of the hyaluronic acid carrier;
- they perform a double mechanism of action on the pathology: a local supplementation of hyaluronic acid and the diacerein inhibition of the collagenolytic activity induced by IL-1β;
- they do not cause local or systemic toxic risks because they do not contain free diacerein, and the total amount of diacerein is negligible in comparison to the dosage authorized in humans.

The process, object of the invention, allows to obtain a non-hydrolyzed diacerein ester with hyaluronic acid, straw-white in colour, without detectable impurities, having the formula

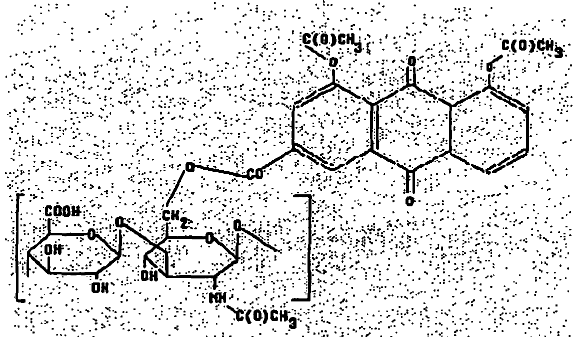

This process envisages protection of the diacerein carboxylic group with N,N-carbodiimidazole (CDI) in accordance with a classic method used to esterify aminoacids (Synthesis 833,1982).

The synthesis of imidazolyl diacerinate (CDIDIAC) occurs by stoichiometric reaction in anhydrous organic solvent at a temperature of 30-40°C. The preferred solvent is dimethylformamide (DMF), but the synthesis can also occur in other aprotic, polar solvents, as DMSO.

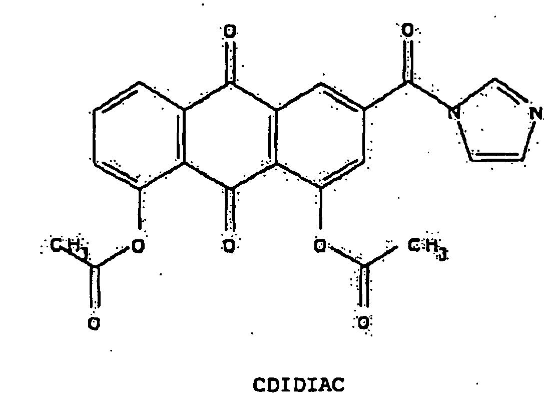

Said reaction does not cause the formation of by-products: the excess of CDI decarboxylates with the liberation of carbon dioxide and imidazole.

The next step of the synthesis, the esterification with hyaluronic acid of the protected diacerein, requires the use of previously salified hyaluronic acid with low quaternary strong bases, such as, for example, the tetrabutylammonium hydroxide (TBAI). Salification makes hyaluronic acid soluble in the same solvent of CDIDIAC, and available to esterify the DIAC by substitution of the imidazole amide.

The salification of the hyaluronic acid with TBAI can be carried out by ionic exchange on resin in accordance with traditional techniques (Butyric and Retinoic Mixed Ester of Hyaluronan, The Journal of Biological Chemistry, Vol. 279, No. 22, Issue of May 28, pp. 23574-23579, 2004; Hyaluronic-acid butyric esters as promising antineoplastic agents in human lung carcinoma: A pre-clinical study, Investigational New Drugs 22: 207-217, 2004.). For example, saturating a sulphonated resin, Amberlite IR-20 type, with a concentrated TBAI solution, washing with water to remove the excess of TBAI, then percolating a dilute solution of hyaluronic acid sodium salt. The tetrabutylammonium salt of the hyaluronic acid (HA-TBA) is separated in solid form by lyophilization and stored in a refrigerator in a container with dehydrating silica gel.

Esterification occurs by the addition of HA-TBA to the CDIDIAC solution in the selected solvent, e.g. DMF, allowing it to react under mechanical stirring at a temperature lower than 40°C for 4-48 hours in an anhydrous environment or made inert with nitrogen.

For the recovery of the ester the reaction mass can be dialysed, for example, with a pH 7 aqueous buffer, and/or ultra filtered, to remove the by-products of the reactions, imidazole and TBAI, then lyophilized.

Alternately, the ester can be isolated by precipitation with a suitable organic solvent, i.e. ethyl alcohol or acetone.

In both cases it is obtained a product with a residual humidity of less than 10%, straw-white in colour and specific viscosity changed or unchanged with respect to that of the starting hyaluronic acid, depending on the diacerein content.

The diacerein content can vary according to the molecular ratio of reagents and by consequence the colour of the final product can vary: it can reach approximately 5% substitution ratio using hyaluronic acid having low molecular weight (LMW, approx. 0.1 10⁶ Dalton) and decrease to 1.5% using hyaluronic acid with a higher molecular weight (HMW, 1.2 10⁶ Dalton). The colour of the latter derivative is a lighter straw-yellow than the former.

The level of esterification of the hyaluronate hydroxy groups depends on many factors, including molecular mass, viscosity, concentration of the HA-TBA solution, as well as the stoichiometric ratio in reaction with the CDIDIAC, and time and temperature used.

### Example 1

### Hyaluronic acid tetrabutylammonium salt (HA-TBA)

The resin (Amberlite IR-20, in acid form CAS 9002-23-7) has a declared capacity of 1.9 eq/L: to salify 1 litre of it, approximately 1,250 mL of tetrabutylammonium hydroxide solution at 40% (TBAI) are necessary. To obtain an efficient exchange using a ratio of 10:1 between the resin sulphonic groups, and the hyaluronic acid carboxylic groups, one litre of activated resin is sufficient for approximately 75-80 g of sodium hyaluronate.

Preparation is carried out on a chromatographic column packed with approximately 0.1 L of resin medium, washed with 0.5 L of demineralized water. The quantity of TBAI, solution at 40% (1.25 L/litre of resin), is percolated and recycled with a pump having a flow of approximately 0.05 - 0.1 volumes of resin/hour for a time corresponding to 3-4 recycles (approximately 2-3 days). Once the cycle is completed, the resin is washed with demineralized water (equal to at least 5-6 volumes of resin) to obtain an eluate with a stable pH between 9.5 - 10. To obtain a more efficient exchange it is appropriate to use a jacketed column, thermostatically controlled at 40°C. Grams 8 of sodium (LMW) HA are dissolved in approximately 2 L of demineralized water (suggested concentration 2-4 g/L of HA of M.W. 0.6 10⁶ Dalton) and the solution is treated on resin column (0.1 L) at a temperature lower than 35°C at a flow of approximately 0.1-0.2 L/h. The column percolate and the washing waters (approximately 0.5 L) are collected and subjected to lyophilization.

Grams 7.5 of the product are obtained having humidity lower than 10%.

### Example 2

### Hyaluronic acid tetrabutylammonim salt (HA-TBA)

Grams 8 of (HMW) sodium hyaluronate are dissolved in approximately 5 L of demineralized water (suggested concentration 1-2 g/L per HA of M.W. 1.2 10⁶ Dalton) and the solution is treated on resin column (0.1 L) at a temperature lower than 35°C at a flow of approximately 0.4 L/h. The percolate from the column and the washing waters (approximately 0.5 L) are collected and are subjected to lyophilization.

Grams 7.2 of the product are obtained having humidity lower than 10%.

### Example 3

### (LMW) Hyaluronic acid diacerein ester

Grams 2.20 of diacerein (-6 mmol) are dissolved in 100 mL of anhydrous dimethylformamide to which, under stirring, 1.30 g (∼ 7 mmol) of N,N-carbonyldiimidazole are added: the mass is left to react at room temperature for 12 hours or overnight in a flask protected from humidity. Then 5 g of (HMW) HA -TBA dissolved in 250 mL of DMF are added and left under stirring for 24-48 hours until the mass of the reaction becomes a transparent and homogeneous red gel. To the residue after sedimentation, 100 mL of pH 7 phosphate buffer are added and transferred into a dialysis bag. PTFE membrane, size 200-400 nominal Dalton. Outer dialysis solution was monitored for colour and changed many times to colourless solution over 48 hours. The dialyzed solution is lyophilized and 5.8 g of product are obtained having humidity lower that 10%.

### Example 4

### (HMW) Hyaluronic acid diacerein ester

In a flask under nitrogen atmosphere 2.2 g of diacerein (~6 mmol) are dissolved in 100 mL of anhydrous DMF and 1.45 g of N,N-carbonyldiimidazole are added thereto under stirring. The mass is left to react at room temperature till complete solution or transparency. Grams 5 of HA-TBA (HMW), previously dissolved in 50 mL of dimethylformamide, are added thereto through a dropping funnel and the reaction mixture, protected from humidity, is kept under agitation for 24-48 hours.

The reaction is stopped by adding under stirring 100 mL of a saturated solution of sodium chloride. The mass is precipitated by addition of about 2 volumes of 96% ethanol and the supernatant is discharged. The residue is washed several times with ethanol at different concentration and finally dried under vacuum.

Grams 5.3 of product are obtained having humidity lower than 10%.

### Pharmaceutical formulations containing the hyaluronic acid diacerein ester

All the preparation must be performed in sterile area with previously sterilised equipment.
a. Milligrams 500 of the (HMW) hyaluronic acid diacerein ester, as prepared in Example 4, are dissolved in 50 mL of saline and kept under stirring for 1 h. The final solution is sterilized by saturated steam at a proper time and temperature, validated by means of F₀, to give an SAL of 10⁻⁶ or better. Then 2 mL of the obtained solution are filled in a vial.
b. Milligrams 500 of the (HMW) hyaluronic acid diacerein ester, as prepared in Example 4, are dissolved in 50 mL pH 7 phosphate buffer 0.01M and kept under stirring for 1 h. The final solution is sterilized by saturated steam at a proper time and temperature, validated by means of F₀, to give an SAL of 10⁻⁶ or better. Then 2 mL of the obtained solution are filled in a vial.
c. Milligrams 500 of the (LMW) hyaluronic acid diacerein ester, as prepared in Example 3, are dissolved in 50 mL of pH 6.5 phosphate buffer 0.01M/glicerol (6:4 v/v) and kept under stirring for 1 h. The final solution is sterilized by saturated steam at a proper time and temperature, validated by means of F₀, to give an SAL of 10⁻⁶ or better. Then 2 mL of the obtained solution are filled in a vial.

### Structural identification of hyaluronic acid diacerein ester

The derivatives of hyaluronic acid and diacerein, prepared according to the process of the invention, have been proved by ¹H and ¹³C NMR performed on the TBA salt in DMSO. The final esters a sodium salts have not enough solubility in DMSO to produce significant NMR signals, whereas the TBA salts have. Due to the low concentration of diacerein in the DMSO solution, it was necessary to accumulate overnight the signals in both ¹H and ¹³C NMR, in order to identify the specific signals of diacerein.

The ¹H NMR spectrum showed a chemical shift of the aromatic signals of diacerein of about 0.5 ppm, from 7.5-8.5 ppm (pure diacerein) to 7.0-8.0 ppm (ester), due to the ester bond between diacerein and hyaluronic acid.

The high noise of signals (due to the overnight accumulation) did not let to see the signals of the acetyl groups so, in order to demonstrate that diacerein was not hydrolyzed by the reaction, the ¹³C NMR was performed.

As expected, the ¹³C NMR spectrum demonstrated that the ¹³C carbon's signal of diacerein's acetyl groups was shifted of about 1 ppm, from 20 ppm (pure diacerein) to 21 ppm (ester).

The above results combined together demonstrated that the diacerein was not hydrolyzed and it was structurally bonded to hyaluronic acid.

### Analytical properties

Solubility > 5mg/mL in water
pH in water: 7.0 - 8.0
Moisture (K.F.): >5%
Identification of diacerein by transacetylation of benzylamine: positive
Free diacerein (HPLC): < 0.01%
Free rhein (HPLC): <0.01%
Substitution ratio: 2-5%

## Claims

1. A process for the preparation of stable esters of diacerein with hyaluronic acid having prolonged anti-inflammatory activity and suitable for intra-articular administration, wherein diacerein, suitably protected in the carboxylic group with a carbodiimidazolyl radical, is made to react with hyaluronic acid, suitably salified with a strong quartenary base, at a temperature lower than 40°C for a period lasting from 4 to 48 hours in aprotic solvent and in the presence of nitrogen and the reaction mass is then submitted to dialysis and lyophilized, giving the desired compound.

2. The process according to claim 1, wherein the hyaluronic acid has a molecular weight of between 100,000 Da and 1,500,000 Da and the diacerein substitution ratio in the ester with the hyaluronic acid is between 0.5 and 5%.

3. Stable esters of diacerein with hyaluronic acid having a prolonged anti-inflammatory activity and suitable for intra-articular administration as prepared according to the process of claim 1 or 2.

4. A pharmaceutical composition with prolonged anti-inflammatory activity suitable for intra-articular administration, which includes a compound as prepared according to claim 3 suitably mixed with a pharmaceutically acceptable vehicle.

## Patentansprüche

1. Verfahren zum Herstellen stabiler Ester von Diacerein mit Hyaluronsäure, die eine anhaltende anti-inflammatorische Aktivität aufweisen und für die intraartikuläre Verabreichung geeignet sind, wobei das Diacerein, das entsprechend an der Carboxylgruppe mit einem Carbodiimidazolyl-Radikal geschützt ist, mit Hyaluronsäure, die entsprechend mit einer starken quaternären Base einer Salzbildung unterzogen wurde, bei einer Temperatur von weniger als 40°C, für eine Zeitdauer von 4 bis 48 Stunden in einem aprotischen Lösungsmittel und in Gegenwart von Stickstoff umgesetzt wird, und wobei die Reaktionsmasse dann einer Dialyse unterzogen und lyophilisiert wird, was die gewünschte Verbindung ergibt.

2. Verfahren nach Anspruch 1, wobei die Hyaluronsäure ein Molekulargewicht zwischen 100000 Da und 1500000 Da aufweist und das Diacerein-Substitutionsverhältnis in dem Ester mit Hyaluronsäure zwischen 0,5 und 5 % liegt.

3. Stabile Ester von Diacerein mit Hyaluronsäure, die eine anhaltende anti-inflammatorische Aktivität aufweisen und für die intraartikuläre Verabreichung geeignet sind, wie sie nach dem Verfahren gemäß Anspruch 1 oder 2 hergestellt werden.

4. Pharmazeutische Zusammensetzung, die eine anhaltende anti-inflammatorische Aktivität aufweist und für die intraartikuläre Verabreichung geeignet ist, welche eine nach Anspruch 3 hergestellte Verbindung einschließt, und die entsprechend mit einem pharmazeutisch geeigneten Träger gemischt wird.

## Revendications

1. Procédé pour la préparation d'esters stables de diacéréine avec de l'acide hyaluronique ayant une activité anti-inflammatoire prolongée et utilisables pour une administration par voie intra-articulaire, dans lequel la diacéréine, convenablement protégée au niveau du groupe carboxylique par un radical carbodiimidazolyle, est mise en réaction avec l'acide hyaluronique convenablement salifié avec une base quaternaire forte, à une température inférieure à 40 °C pendant une période allant de 4 à 48 heures dans un solvant aprotique et en présence d'azote et la masse réactionnelle est ensuite soumise à dialyse et lyophilisée, pour donner le composé souhaité.

2. Procédé selon la revendication 1, dans lequel l'acide hyaluronique a un poids moléculaire compris entre 100 000 et 1 500 000 Da et le taux de substitution de la diacéréine dans l'ester par l'acide hyaluronique est compris entre 0,5 et 5 %.

3. Esters stables de diacéréine avec de l'acide hyaluronique ayant une activité anti-inflammatoire prolongée et utilisables pour une administration par voie intra-articulaire, préparés selon le procédé des revendications 1 ou 2.

4. Composition pharmaceutique ayant une activité anti-inflammatoire prolongée et utilisable pour une administration par voie intra-articulaire, qui comprend un composé préparé selon la revendication 3 et convenablement mélangé avec un véhicule pharmaceutiquement acceptable.
